(19) Europäisches Patentamt
European Patent Office
Office européen des brevets

(11) Publication number: **0 248 554 B1**

(12) # EUROPEAN PATENT SPECIFICATION

(45) Date of publication of patent specification: **03.02.93**

(51) Int. Cl.5: **C07D 239/84**, A01N 43/54

(21) Application number: **87304292.3**

(22) Date of filing: **14.05.87**

The file contains technical information submitted after the application was filed and not included in this specification

(54) **Heterocyclic compounds.**

(30) Priority: **30.05.86 GB 8613200**
**25.02.87 GB 8704390**

(43) Date of publication of application:
**09.12.87 Bulletin 87/50**

(45) Publication of the grant of the patent:
**03.02.93 Bulletin 93/05**

(84) Designated Contracting States:
**AT BE CH DE ES FR GB GR IT LI LU NL SE**

(56) References cited:
**EP-A- 0 044 163**

(73) Proprietor: **IMPERIAL CHEMICAL INDUSTRIES PLC**
**Imperial Chemical House, Millbank**
**London SW1P 3JF(GB)**

(72) Inventor: **Barton, John Edward Duncan**
**96 Kendrick Road**
**Reading, Berkshire(GB)**
Inventor: **Collins, David John**
**10 Ardwell Close**
**Crowthorne, Berkshire(GB)**
Inventor: **Slater, John Walter**
**27 Longleat Gardens**
**Maidenhead, Berkshire(GB)**

(74) Representative: **Hardman, Carol Pauline**
**ICI Group Patents Services Dept. PO Box 6**
**Shire Park Bessemer Road**
**Welwyn Garden City Herts, AL7 1HD(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to the use of certain herbicidal compounds, in the control of weeds in rice.

Published European Patent Application 0044163 discloses herbicidal compounds of formula (I)

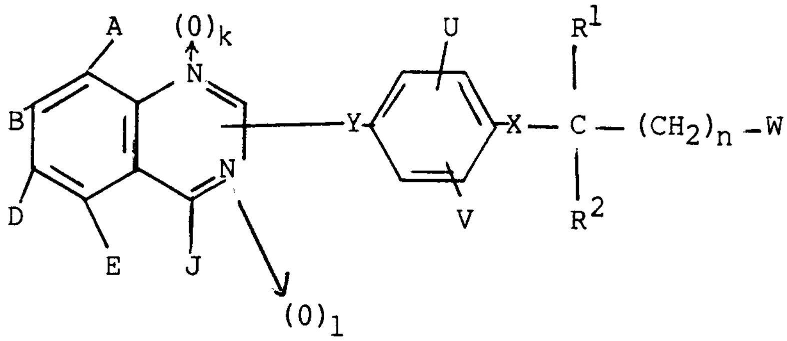

(I)

wherein A,B,D,E,J,U and V are independently selected from hydrogen and various specified organic substituents;

$R^1$ and $R^2$ are selected from hydrogen or various specified organic groups;

W is cyano, thiocarbamoxy,

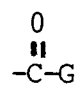

or $CH_2Z$ where G is selected from various specified organic groups including hydroxy and OM where M is a cation; and Z is selected from various specified organic groups;

X is oxygen or sulphur;

Y is oxygen, sulphur or-NR where R is hydrogen or various specified organic groups;

k and l are independently 0 or 1; and

n is 0, 1 or 2.

The applicants have found a group of compounds some of which fall within the scope of formula (I) above, which show a very useful selectivity in rice crops.

According to one aspect of the present invention there is provided a method for selectively controlling the growth of weeds in rice which process comprises applying to the rice or to the growth medium of the rice, a compound of formula (IIA):

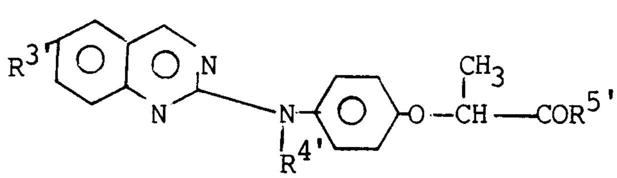

or an R-enantiomer thereof wherein $R^{3'}$ is halogen, $CF_3$ or methyl; $R^{4'}$ is methyl or ethyl; and $R^{5'}$ is $OR^6$ or $NHSO_2R^7$ wherein $R^6$ is hydrogen; $C_{1-10}$ alkyl, $C_{2-10}$ alkenyl or $C_{2-10}$ alkynyl, any of which may be optionally substituted by phenyl, $C_{1-10}$ alkoxy, phenyl $C_{1-10}$ alkoxy, phenoxy, cycloalkyl of up to 10 carbon atoms or tri$C_{1-10}$alkylsilyl; $C_{3-6}$ cycloalkyl; phenyl; p-nitrophenyl, p-chlorophenyl; 2,4-dichlorophenyl; or a cation and $R^7$ is $C_{1-10}$ alkyl or halo $C_{1-10}$ alkyl; in an amount sufficient to severely damage or kill the weeds but insufficient to damage the rice substantially.

As used herein the term "aryl" includes phenyl.

Suitable halogen groups $R^{3'}$ include fluorine, chlorine and bromine, preferably chlorine.

Preferably $R^{4'}$ is methyl.

When $R^6$ is an optionally substituted $C_{1-10}$ alkyl, an optionally substituted $C_{2-10}$ alkenyl or an optionally substituted $C_{2-10}$ alkynyl group, it may have a straight or branched chain arrangement. Suitably these groups $R^6$ contain up to six carbon atoms, for example up to four carbon atoms.

Particular examples of optional for group $R^6$ substituents include $C_{1-4}$ alkoxy such as methoxy, benzyloxy, phenyl and trimethylsilyl.

Suitable groups $R^6$ include hydrogen, methyl, ethyl, propyl, and iso-propyl, n-butyl, n-pentyl, 2-(methyl)-butyl, 2-(methyl)propyl, 1-(methyl)-propyl, 1-(ethyl)-propyl, 2,2,-(dimethyl)propyl, 1-(methyl)butyl, cyclohexyl-methyl, 3-(methyl)butyl 1,3-(dimethyl)propyl, prop-2-enyl or prop-2-ynyl, 2-(methoxy)ethyl, 1-(methyl)-2-(methoxy)ethyl, 2-(methoxy)propyl, benzyl, 2-(benzyloxy)ethyl, para-nitro phenyl and methyl-trimethylsilyl.

Preferably $R^6$ is $C_{1-6}$ alkyl such as ethyl or 2,2-(dimethyl)propyl.

Suitable cations for $R^6$ are agriculturally acceptable cations such as sodium, potassium or ammonium ions.

Examples of ammonium ions are ions of formula $NR^8R^9R^{10}R^{11}$ where $R^8$, $R^9$, $R^{10}$ and $R^{11}$ are independently selected from hydrogen or $C_{1-10}$ alkyl optionally substituted with for example hydroxy. Suitably where any of $R^8$, $R^9$, $R^{10}$ and $R^{11}$ are optionally substituted alkyl, they contain from 1 to 4 carbon atoms.

Particular examples of $R^8$, $R^9$, $R^{10}$ and $R^{11}$, are hydrogen, ethyl and 2-hydroxyethyl.

Suitably $R^7$ is $C_{1-6}$ alkyl such as methyl ethyl or propyl in particular methyl.

When $R^7$ is halo $C_{1-10}$ alkyl, it is suitably trifluoromethyl.

Compounds of formula (IIA) contain an asymetric carbon atom and therefore exist in isomeric form. Unless otherwise stated in the provisos above, the invention covers the use of all isomeric forms and mixtures thereof including racemic mixtures.

As used herein the expression "In the form its R-enantiomer" means that the compound contains more than 50% of the R-enantiomer preferably more than 75% of the R-enantiomer and more preferably more than 90% of the R-enantiomer. In the most preferred embodiment, the compound is substantially free of the S-enantiomer.

Novel compounds of formula (IIA) are those of formula (II)

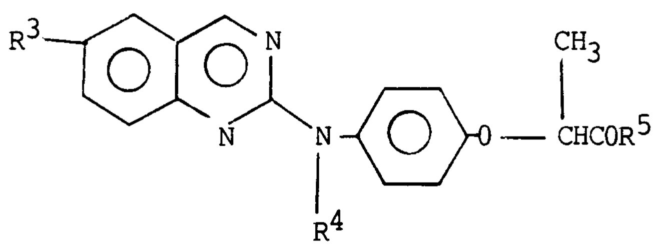

(II)

and R-enantiomers thereof;

wherein $R^3$ is halogen, $CF_3$ or methyl;

$R^4$ is methyl or ethyl; and

$R^5$ is $OR^6$ or $-NHSO_2R^7$ wherein $R^6$ are $R^7$ are as hereinbefore defined; provided that when $R^3$ is chloro, $R^4$ is methyl and $R^5$ is ethoxy, or $R^3$ is bromo, $R^4$ is methyl and $R^5$ is methoxy, the compound is in the form of its R-enantiomer.

Examples of compounds of formula (II) are set out in Table I.

TABLE 1

| COMPOUND NO | $R^3$ | $R^4$ | $R^5$ | CHARACTERISING DATA |
|---|---|---|---|---|
| 1* | -Cl | $-CH_3$ | $-O-CH_2CH_3$ | Gum: Elemental Analysis:<br>C H N<br>calc 62.25 5.23 10.89<br>found 61.10 5.33 10.64 |
| 2* | -F | $-CH_3$ | $-O-CH_2CH_3$ | m.p 74.3-75.9°C<br>Elemental Analysis:<br>C H N<br>calc 65.02 5.47 11.38<br>found 63.95 5.61 11.05 |
| 3 | -Cl | $-CH_3$ | $-O-(CH_2)_2CH_3$ | (oil) NMR δ 8.88s(1); δ 7.60m (3 quinazoline H); 7.24 + 6.94 both d(2), 4.78 q (1); 4.16m (2), 3.58 s (3); 1.68m (3 propyl H); 0.92 t(3) |
| 4 | -Cl | $-CH_3$ | $-O-CH(CH_3)_2$ | NMR: δ 8.88s (1); δ 7.60m (3 quinazoline H); 7.24d (2) + δ6.92d (2); 5.12m (1); 4.72q (1); 3.58s (3); 1.62d (3); 1.30 and 1.24, both d(3) (oil) |

TABLE 1 CONTINUED

| COMPOUND NO | $R^3$ | $R^4$ | $R^5$ | CHARACTERISING DATA |
|---|---|---|---|---|
| 5 | -Cl | $-CH_2CH_3$ | $-O-CH_2CH_3$ | Elemental Analysis: C / H / N; calc 63.07 / 5.55 / 10.51; found 62.87 / 5.54 / 10.10; m.p 71-72°C |
| 6 | $-CH_3$ | $-CH_3$ | $-O-CH_2CH_3$ | (oil) Elemental Analysis: C / H / N; calc 69.02 / 6.34 / 11.50; found 69.02 / 6.61 / 11.46 |
| 7 | -F | $-CH_3$ | -OH | m.p 172.3-175.0°C |
| 8 | -F | $-CH_3$ | $-O-CH_2CH_3$ | NMR δ 8.90s (1); 7.6m (1); 7.44m (1); 7.24m (3); 6.92d (2); 4.75q (1); 4.25 q (2); 3.57s (3); 1.63d (3); 1.28t (3) (gum) |
| 9 | -F | $-CH_3$ | $-O-CH_3$ | NMR δ 8.89s (1); 7.6m (1); 7.44m (1); 7.24m (3); 6.92d (2); δ 4.77q (1); 3.77s (3); 3.57s (3); 1.63d (3) (gum) |

TABLE 1 CONTINUED

| COMPOUND NO | $R^3$ | $R^4$ | $R^5$ | CHARACTERISING DATA |
|---|---|---|---|---|
| 10 | $-CF_3$ | $-CH_3$ | $-O-CH_2CH_3$ | (Gum) Elemental Analysis:<br>C H N<br>calc 60.14 4.81 10.02<br>found 59.70 4.97 9.89<br><br>NMR δ 9.00s (1); δ 7.94s, br (1); δ 7.75m (2); δ 7.32d (2); δ 6.97d (2); δ 4.78q (1); δ 4.27q (2); δ 3.63s (3); δ 1.64d (3); δ 1.29t (3) |
| 11 | -Cl | $-CH_3$ | -OH | m.p 157.5-159.4°C |
| 12 | -Br | $-CH_3$ | -OH | (Glass)<br>Elemental Analysis:<br>C H N<br>calc 53.74 4.01 10.45<br>found 53.57 4.24 9.96<br><br>NMR δ 8.88s (1); 8.10 broad OH (1); 7.78 to 7.08 5 peaks from 3 quinazoline H; 7.24 + 6.96 both d (2); 4.80q (1); 3.57s (3); 1.64d (3) |

TABLE 1 CONTINUED

| COMPOUND NO | $R^3$ | $R^4$ | $R^5$ | CHARACTERISING DATA |
|---|---|---|---|---|
| 13 | -Br | $-CH_3$ | $-O-CH_2CH_3$ | Oil Elemental Analysis<br>C H N<br>calc 55.82 4.68 9.77<br>found 55.32 4.84 9.85 |
| 14 | -Cl | $-CH_3$ | $-O-CH_3$ | NMR δ 8.9s (1); 7.59m (3); 7.25 + 6.95 both d (2); 4.75q (1); 3.79s (3); 3.59s (1); 1.64 d (3)<br>Gum |
| 15* | Cl | $CH_3$ | $-O-(CH_2)_3CH_3$ | oil initialy<br>NMR δ 8.9s (1); 7.6m (3); 7.1q (4); 4.75q (1); 4.2m (2); 3.6s (3); 1.62m (s); 1.35m (2); 0.9t (3) |
| 16* | Cl | $CH_3$ | $-O-CH(CH_3)CH_2CH_3$ | oil initialy<br>NMR δ 8.9s (1); 7.6m (3); 7.1q (4); 4.95m (1); 4.75q (1); 3.6s (3); 1.6m (s); 1.2dd (3); 0.9dt (3). |
| 17* | Cl | $CH_3$ | $-O-CH_2C(CH_3)_3$ | oil initialy<br>NMR δ 8.85s (1); 7.6m (3); 7.1q (4); 4.8q (1); 3.9dd (2); 3.59s (3); 1.62d (3); 0.9s (9). |

TABLE 1 CONTINUED

| COMPOUND NO | $R^3$ | $R^4$ | $R^5$ | CHARACTERISING DATA |
|---|---|---|---|---|
| 18* | Cl | $CH_3$ | $-O-CH_2CH=CH_2$ | oil initialy NMR δ 8.85s (1); 7.6m (3); 7.1q (4); 5.9m (1); 5.3m (2); 4.8q (1); 4.7m (2); 3.6s (3); 1.62d (3) |
| 19* | Cl | $CH_3$ | $-OH$ | NMR δ 9.02s (1); 7.9s (1); 7.65 (1); 7.48d (1); 7.0q (4); 4.79q (1); 3.41s (3); 1.45d (3) |
| 20* | Cl | $CH_3$ | $-O-CH_2C{\equiv}CH$ | oil initialy NMR δ 8.8s (1); 7.5m (3); 6.95q (4); 4.70m (3); 3.5s (3); 2.44m (1); 1.59d (3) |
| 21* | Cl | $CH_3$ | $-O-(CH_2)_2OCH_3$ | oil initially NMR δ 8.78s (1); 7.5m (3); 7.0q (4); 4.7q (1); 4.27m (2); 3.54m (2); 3.50s (3); 3.28s (3); 1.57d (3) |
| 22* | Cl | $CH_3$ | $-O-CH_2-$ 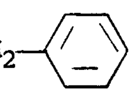 | oil initially NMR δ 8.86s (1); 7.57m (3); 7.33m (s); 7.04q (4); 5.21s (2); 4.8q (1); 3.58s (3); 1.64d (3) |
| 23* | Cl | $CH_3$ | $-O-(CH_2)_2-O-$ $CH_2$ 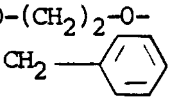 | oil initially NMR δ 8.85s (1); 8.0d (1); 7.6m (3); |

TABLE 1 CONTINUED

| COMPOUND NO | $R^3$ | $R^4$ | $R^5$ | CHARACTERISING DATA |
|---|---|---|---|---|
| | | | | 7.1-7.56m (7); 4.8q (1); 4.54d (3); 4.4m (1); 3.69m (1); 3.55d (3); 1.66d (3) |
| 24* | Cl | $CH_3$ | 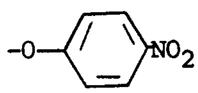 | oil initially<br>NMRδ 8.88s (1); 8.24d (2); 7.6m (3); 7.28q (4); 7.01d (2); 5.01q (1); 3.6s (3); 1.80d (3) |
| 25* | Cl | $CH_3$ | $-\underset{H}{N}SO_2CH_3$ | yellow solid<br>NMRδ 8.87s (1); 7.60m (3); 7.12 q (4); 4.79q (1); 3.6s (3); 3.3s (3); 1.61d (3) |
| 26* | Cl | $CH_3$ | $\overset{\oplus}{\underset{H}{N}}(CH_2CH_2OH)_3$ | yellow solid: m.p. = 140-142°C<br><br>C H N<br>calc 56.85 6.18 11.05<br>found 56.03 6.15 11.91 |
| 27* | Cl | $CH_3$ | $-O-CH_2CH(CH_3)_2$ | yellow oil<br>NMRδ 8.84s (1); 7.56m (3); 7.04q (4); 4.75q (1); 3.93m (2); 3.58s (3); 1.92m (1); 1.64d (3); 0.92d (6) |

TABLE 1 CONTINUED

| COMPOUND NO | $R^3$ | $R^4$ | $R^5$ | CHARACTERISING DATA |
|---|---|---|---|---|
| 28* | Cl | $CH_3$ | $-O-(CH_2)_4CH_3$ | yellow oil<br>NMR δ 8.79s (1); 7.51m (3); 7.0q (4); 4.68m (1); 4.13m (1); 3.94m (1); 3.50s (3); 1.55m (3); 1.0-1.7m (3); 0.82m (6) |
| 29* | Cl | $CH_3$ | $CH_3$<br>\|<br>$-O-CH(CH_2)_2CH_3$ | yellow oil<br>NMR δ 8.79s (1); 7.49m (3); 7.0q (4); 4.92m (1); 4.64q (1); 3.50s (3); 1.54d (3); 1.6-1.16m (4); 1.15dd (3); 0.82dt (3) |
| 30* | Cl | $CH_3$ | $CH_3$<br>\|<br>$-O-CH_2CH-CH_2CH_3$ | yellow oil<br>NMR δ 8.86s (1); 7.58m (3); 7.08q (4); 4.68q (1); 4.04m (2); 3.58s (3); 1.72m (1); 1.64d (3); 1.36m (1); 1.16m (1); 0.88m (6) |
| 31* | Cl | $CH_3$ | $-O-(CH_2)_2-CH-$<br>$-(CH_3)_2$ | yellow oil<br>NMR δ 8.79s (1); 7.5m (3); 6.98q (4); 4.66q (1); 4.12t (2); 3.50s (3); 1.55d (3); 1.44m (3); 0.82m (6) |

TABLE 1 CONTINUED

| COMPOUND NO | $R^3$ | $R^4$ | $R^5$ | CHARACTERISING DATA |
|---|---|---|---|---|
| 32* | Cl | $CH_3$ | $CH_3$<br>$-O-CH-CH(CH_3)_2$ | yellow oil<br>NMR δ 8.82s (1); 7.54m (3); 7.02q (4); 4.58q (1); 3.51s (3); 1.72q (2); 1.54d (3); 1.36d (6); 0.79d (3) |
| 33* | Cl | $CH_3$ | $-O-CH_2-$ 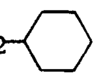 | yellow oil<br>NMR δ 8.86s (1); 7.59m (3); 7.08q (4); 4.76q (1); 3.99m (2); 3.58s (3); 1.78-1.52m (9); 1.30-0.82m (5) |
| 34* | Cl | $CH_3$ | $-O-CH(CH_3)_2$ | yellow oil<br>NMR δ 8.80s (1); 7.52m (3); 6.96q (4); 5.02m (1); 4.60q (1); 3.51s (3); 1.56d (3); 1.19dd (6) |
| 35* | Cl | $CH_3$ | $CH_3$<br>$-O-CH-CH_2OCH_3$ | yellow oil<br>NMR δ 8.85s (1); 7.59m (3); 7.08q (4); 5.19m (1); 4.75q (1); 3.58s (3); 3.47-3.28m (5); 1.63d (3); 1.25dd (3) |
| 36* | Cl | $CH_3$ | $-O-CH_2-CH-CH_3$<br>$OCH_3$ | |

TABLE 1 CONTINUED

| COMPOUND NO | $R^3$ | $R^4$ | $R^5$ | CHARACTERISING DATA |
|---|---|---|---|---|
| 37* | Cl | $CH_3$ | $OCH(CH_2CH_3)_2$ | yellow oil NMR δ 8.86s (1); 7.58m (3); 7.08q (4); 4.48m (1); 4.76q (1); 3.57s (3); 1.65d (3); 1.56m (4); 0.90t (3); 0.79t (3) |
| 38* | Cl | $CH_3$ | $OCH_2Si(CH_3)_3$ | |

Compounds marked * are in the form of the R-enantiomer substantially free of the S-enantiomer.

Compounds of formula (II) can be prepared by methods analogous to those described in EP-A-0044163.

For example compounds of formula (II) can be prepared by reacting a compound of formula (III)

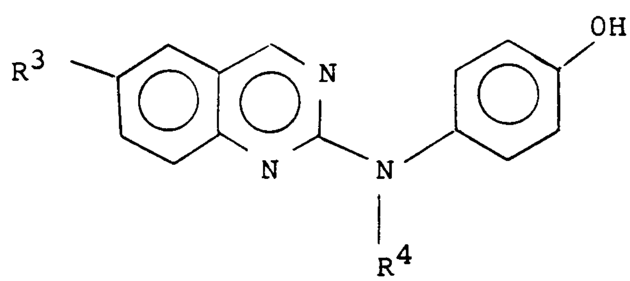

(III)

where $R^3$ and $R^4$ are as defined in relation to formula (II), with a compound of formula (IV)

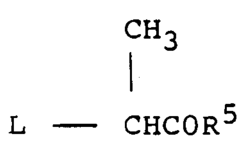

(IV)

where $R^5$ is as defined in relation to formula (II) and L is a leaving group.

Suitable leaving groups L include mesylate, tosylate and halogen. The reaction is suitably carried out in the presence of a strong base such as sodium hydride and in an inert organic solvent such as toluene.

When a compound of formula (II) in enantiomeric form is required, the isomers may be separated after preparation by conventional methods. Alternatively an optionally active compound of formula (IV) for

example L (-) ethyl lactate mesylate may be employed in the reaction, to yield the appropriate isomeric form of the compound of formula (II) directly.

Compounds of formula (IV) are either known compounds or can be prepared from known compounds by known methods.

Compounds of formula (III) can be prepared by reactions carried out in accordance with Scheme A below.

Scheme A

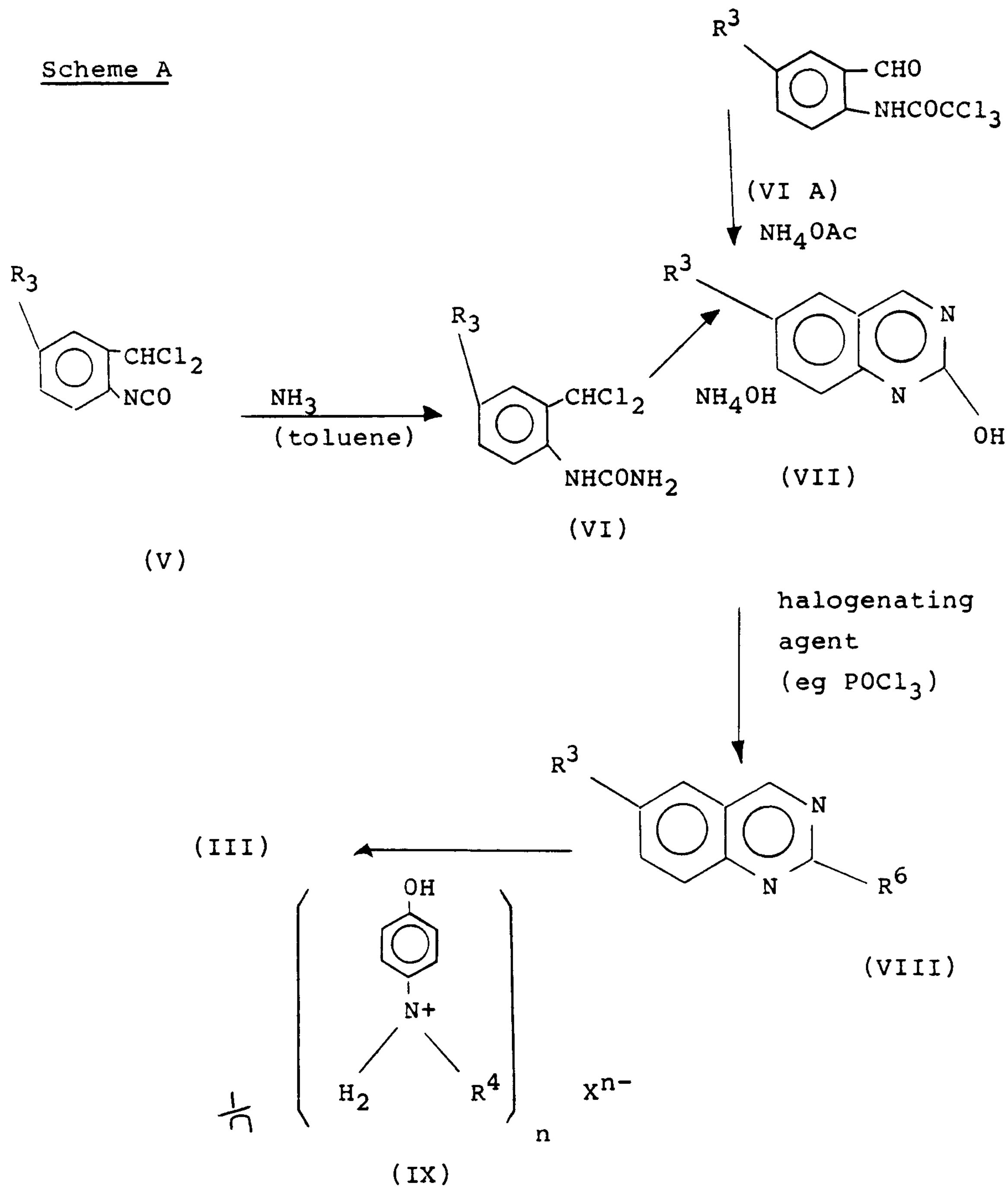


(V) (VI) (VII) (VIII) (III) (IX)

wherein $R^3$ and $R^4$ are as hereinbefore defined, $R^6$ is halogen such as chlorine and X is a cation of valency n, for example sulphate where n is 2.

The reactions are carried out under conventional conditions. Compounds of formula (V) and (IX) are known compounds or can be prepared from known compounds by conventional methods. Compounds of formula (VI A) can be prepared by conventional methods depending upon the nature of the group $R^3$.

Alternatively compounds of formula (II) can be prepared by reacting a compound of formula (VIII) as

hereinbefore defined with a compound of formula (X)

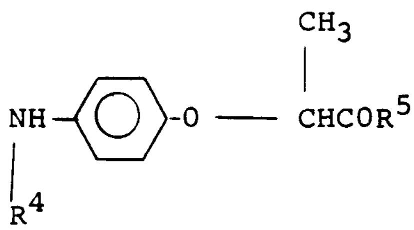

(X)

wherein $R^4$ and $R^5$ are as defined in relation to formula (II).

The reaction is suitably carried out in an organic solvent such as diglyme. If the compound of formula (II) is required in enantiomeric form, the compound of formula (X) is suitably in the form of the single appropriate isomer. Compounds of formula (X) are either known compounds or can be prepared from known compounds by known methods. A preparation for an enantiomeric form of the compound of formula (X) is shown in Scheme B below.

Scheme B

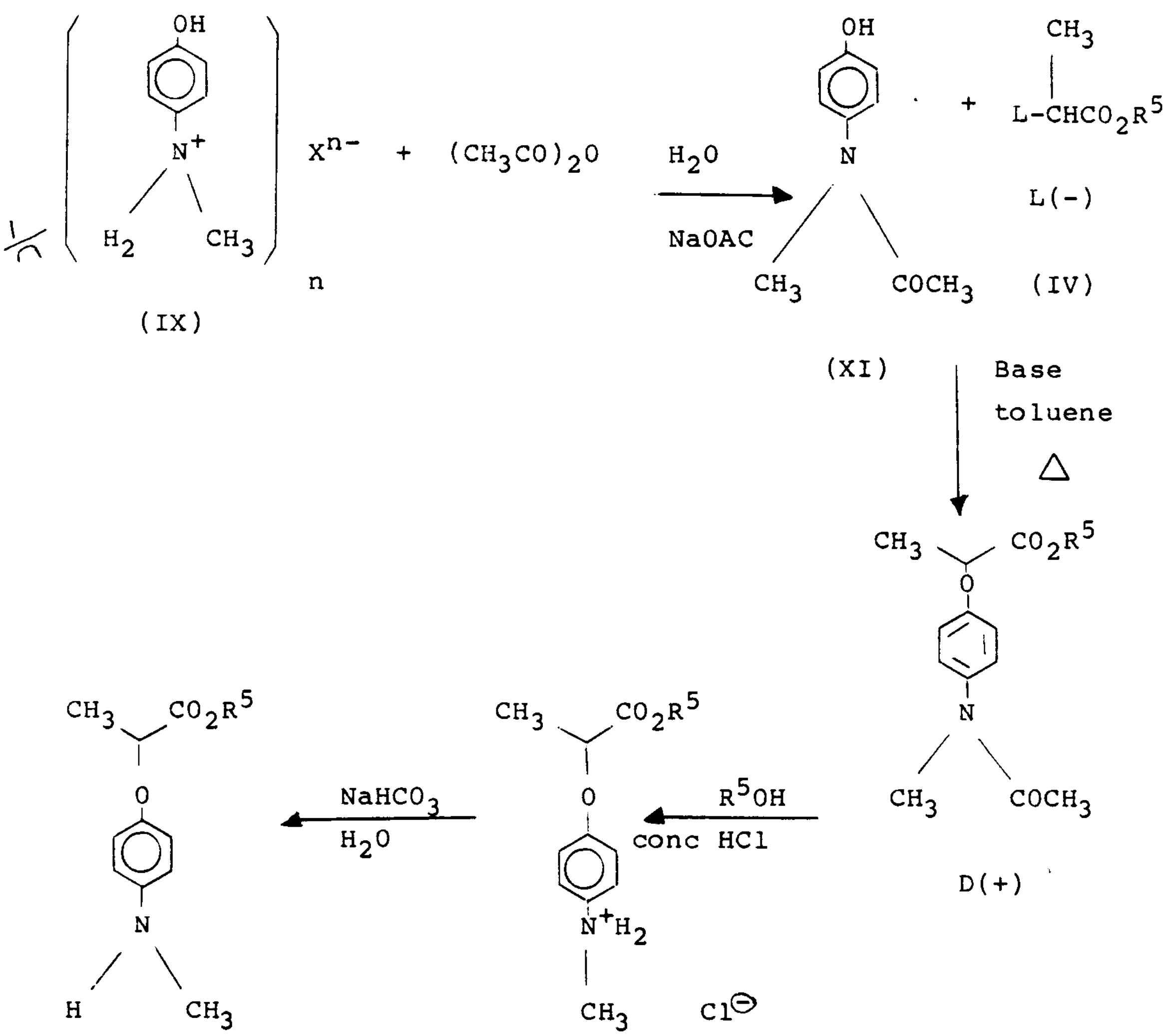


(IX) (XI) L(-) (IV) D(+)

(X) D(+)

Compounds of formula (II) where $COR^5$ is an ester group of formula $-CO_2R^6$ can be converted to compounds of formula (II) where $R^6$ is hydrogen by conventional deesterification methods, for example by acid or base hydrolysis. Similarly compounds of formula (II) where $R^5$ is $OR^6$ and $R^6$ is hydrogen may be esterified to convert $R^6$ to another such group.

Compounds of formula (II) where $COR^5$ is an ester group can be converted to a different such ester by standard trans-esterification techniques for example by reacting the compound of formula (II) with an appropriate alcohol is the presence of an acid such as concentrated sulphuric acid. Where the alcohol is sensitive to acid, trans-esterfication can be carried out in two steps, by first converting one ester to the acid and subsequently reacting the acid with the alcohol under appropriate conditions for example in the presence of a tertiary base and a dehydrating agent such as dicyclohexylcarbodiimide (DCC).

Compounds of formula (II) where $R^5$ is $OR^6$ and $R^6$ is a cation can be prepared by reacting the compound of formula (II) where $COR^5$ is an acid group with an appropriate base.

Compounds of formula (II) where $R^5$ is $-NHSO_2R^7$ can be prepared by reacting a compound of formula (II) where $COR^5$ is a displaceable ester group with a compound of formula $H_2N-SO_2R^7$ where $R^7$ is as defined in relation to formula (II) in the presence of a base. The reaction is carried out using conventional methods for example in an inert organic solvent such as tetrahydrofuran at moderate temperatures for example at room temperature. Suitable displaceable ester groups include para-nitrophenyl ester. Suitable bases include strong bases such as sodium hydride.

Further Examples of compounds of formula (IIA) include the compounds listed in Table 2.

TABLE 2

| COMPOUND NO | $R^{3'}$ | $R^{4'}$ | $R^{5'}$ |
|---|---|---|---|
| 39 | Cl | $CH_3$ | $OCH_2CH_3$ |
| 40 | Br | $CH_3$ | $OCH_3$ |

The compounds of formulae (IIA) may be applied directly to the plant (post-emergence application) or to the soil before the emergence of the plant (pre-emergence application). However, the compounds are, in general, more effective when applied to the plant post-emergence.

The compounds of formulae (IIA) may be used on their own to inhibit the growth of, severely damage, or kill plants but are preferably used in the form of a composition comprising a compound of the invention in ad-mixture with a carrier comprising a solid or liquid diluent.

Compositions containing compounds of formula (IIA) include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, eg, kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type. The cationic agents are, for example, quaternary ammonium compounds (eg cetyltrimethylammonium bromide). Suitable anionic agents are soaps; salts or aliphatic mono ester of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixutre of the sodium salts of diisopropyl and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol or octyl-cresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or

an organic solvent optionally containing wetting or dispersing agent(s) and then, when organic solvents are used, adding the mixture so obtained to water optionally containing wetting or dispersing agent(s). Suitable organic solvents include, for example, ethylene di-chloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then diluted with water before use. The concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20-90%, preferably 20-70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprising the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties too, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited, the formulations selected for use and whether the compound is to be applied for foliage or root uptake. As a general guide, however, an application rate of from 0.005 to 20 kilograms per hectare is suitable while from 0.1 to 10 kilograms per hectare may be preferred.

The compositions may comprise, in addition to one or more compounds of formula (IIA), one or more compounds not of formula (IIA) but which possess biological activity. Accordingly a herbicidal composition comprising a mixture of at least one herbicidal compound of formula (IIA) as hereinbefore defined with at least one other herbicide may be provided.

The other herbicide may be any herbicide not having the formula (IIA). It will generally be a herbicide having a complementary action in the particular application. For example it may be desirable in certain circumstances to use the compound of formula (IIA) in admixture with a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (bentazon);

B. hormone herbicides, particularly the phenoxy alkanoic acids such as 4-chloro-2-methylphenoxy acetic acid (MCPA), 2-(2,4-dichlorophenoxy)propionic acid (dichlorprop), 2,4,5-trichlorophenoxyacetic acid (2,4,5-T), 4-(4-chloro-2-methylphenoxy)butyric acid (MCPB), 2,4-dichlorophenoxyacetic acid (2,4-D), 4-(2,4-dichlorophenoxy)butyric acid (2,4-DB), 2-(4-chloro-2-methylphenoxy)propionic acid (mecoprop), and their derivatives (eg. salts, esters and amides);

C. 3-[4-(4-halophenoxy)phenyl]-1,1-dialkylureas such as 3-[4-(4-chlorophenoxy)phenyl]-1,1-dimethylurea.

D. Dinitrophenols and their derivatives (eg. acetates) such as 2-methyl-4,6-dinitrophenol (DNOC), 2-t-butyl-4,6-dinitrophenol (dinoterb), 2-secbutyl-4,6-dinitrophenol (dinoseb) and its ester, dinoseb acetate;

E. dinitroaniline herbicides such as N',N'-diethyl-2,6-dinitro-4-trifluoromethyl-m-phenylenediamine (dinitramine), 2,6-dinitro-N,N-dipropyl-4-trifluoromethylaniline (trifluralin) and 4-methylsulphonyl-2,6-dinitro-N,N-dipropylaniline (nitralin);

F. phenylurea herbicides such as N'-(3,4-dichlorophenyl)-N,N-dimethylurea (diuron) and N,N-dimethyl-N'-[3-(trifluoromethyl)phenyl]urea (flumeturon);

G. phenylcarbamoyloxyphenylcarbamates such as 3-[methoxy carbonylamino]phenyl (3-methylphenyl)-carbamate (phenmedipham) and 3-[ethoxycarbonylamino]phenyl phenylcarbamate (desmedipham);

H. 2-phenylpyridazin-3-ones such as 5-amino-4-chloro-2-phenylpyridazin-3-one (pyrazon);

I. uracil herbicides such as 3-cyclohexyl-5,6-trimethyleneuracil (lenacil), 5-bromo-3-sec-butyl-6-methyl-uracil (bromacil) and 3-t-butyl-5-chloro-6-methyl-uracil (terbacil);

J. triazine herbicides such as 2-chloro-4-ethylamino-6-(i-propylamino)-1,3,5-triazine (atrazine), 2-chloro-4,6-di(ethylamino)-1,3,5-triazine (simazine) and 2-azido-4-(i-propylamino)-6-methylthio-1,3,5-triazine (aziprotryne);

K. 1-alkoxy-1-alkyl-3-phenylurea herbicides such as 3-(3,4-dichlorophenyl)-1-methoxy-1-methylurea

(linuron), 3-(4-chlorophenyl)-1-methoxy-1-methylurea (monolinuron), 3-(4-bromo-4-chlorophenyl)-1-methoxy-1-methylurea (chlorobromuron).

L. thiolcarbamate herbicides such as S-propyl dipropylthiocarbamate (vernolate);

M. 1,2,4-triazin-5-one herbicides such as 4-amino-4,5-dihydro-3-methyl-6-phenyl-1,2,4-triazine-5-one (metamitron) and 4-amino-6-t-butyl-4,5-dihydro-3-methylthio-1,3,4-triazin-5-one (metribuzin);

N. benzoic acid herbicides such as 2,3,6-trichlorobenzoic acid (2,3,6-TBA), 3,6-dichloro-2-methoxybenzoic acid (dicamba) and 3-amino-2,5-dichlorobenzoic acid (chloramben);

O. anilide herbicides such as N-butoxymethyl-chloro-2′,6′-diethylacetanilide (butachlor), the corresponding N-methoxy compound (alachlor), the corresponding N-i-propyl compound (propachlor), 3′,4′-dichloropropionanilide (propanil) and 2-chloro-N-[pyrazol-1-ylmethyl]acet-2′-6′-xylidide (metazachlor);

P. dihalobenzonitrile herbicides such as 2,6-dichlorobenzonitrile (dichlobenil), 3,5-dibromo-4-hydroxybenzonitrile (bromoxynil) and 3,5-diiodo-4-hydroxybenzonitrile (ioxynil);

Q. haloalkanoic herbicides such as 2,2-dichloropropionic acid (dalapon), trichloroacetic acid (TCA) and salts thereof;

R. diphenylether herbicides such as 4-nitrophenyl 2-nitro-4-trifluoromethylphenyl ether (fluorodifen), methyl 5-(2,4-dichlorophenoxy)-2-nitrobenzoate (bifenox), 2-nitro-5-(2-chloro-4-trifluoromethylphenoxy)-benzoic acid (acifluorfen) and salts and esters thereof, 2-chloro-4-trifluoromethylphenyl 3-ethoxy-4-nitrophenyl ether (oxyfluorfen) and 5-(2-chloro-4-(trifluoromethyl)phenoxy)-N-(methylsulfonyl)-2-nitrobenzamide (fomesafen); and

S. phenoxyphenoxypropionate herbicides such as 2-(4-(4′-trifluoromethylphenoxy)-phenoxy)-propionic acid methylester (trifop-methyl), 2-(4-((5-trifluoromethyl)-2-(pyridinyl)oxy)phenoxypropanoic acid (fluazifop) and esters thereof, 2-(4-((3-chloro-5-trifluoromethyl)-2-pyridinyl)oxy)phenoxy)propanoic acid (haloxyfop) and esters thereof, 2-(4-((6-chloro-2-quinoxalinyl)oxy)phenoxypropanoic acid (xylofop) and esters thereof; and

T. cyclohexanedione herbicides such as 2,2-dimethyl-4,6-dioxo-5-(1-((2-propenyloxy)amino)-butylidine) cyclohexane carboxylic acid (alloxydim) and salts thereof, 2-(1-ethoxyimino)butyl-5-(2-(ethylthio)propyl)-3-hydroxy-2-cyclohexen-1-one (sethoxydim), 2-(1-(3-chloroallyloxyimino)butyl)-5-(2-ethylthiopropyl)-3-hydroxy cyclohex-2-enone (cloproxydim), 2-(1-ethoxyimino)butyl)-3-hydroxy-5-thian-3-yl cyclohex-2-enone (cycloxydim); and

U. sulfonyl urea herbicides such as 2-chloro-N-(4-methoxy-6-methyl-1,3,5-triazin-2-yl)-aminocarbonyl) benzenesulphonamide (chlorosulfuron), methyl 2-((((4,6-dimethyl-2-pyrimidinyl)amino)carbonyl)amino)-sulphonylbenzoic acid (sulfometuron), 2-(((3-(4-methoxy-6-methyl-1,3,5-triazin-2-yl)carbonyl)amino)-sulphonyl)benzoic acid (metsulfuron), 2-(((4,6-dimethoxypyrimidin-2-yl)amino carbonyl) aminosulphonylmethyl) benzoic acid (benzsulfuron) and esters thereof

V. imidazolidinone herbicides such as 2-(4,5-dihydro-4-isopropyl-4-methyl-5-oxoimidazol-2-yl)quinoline-3-carboxylic acid (imazaquin), methyl 6-(4-isopropyl-4-methyl-5-oxo-2-imidazolin-2-yl)-m-toluate and p-toluate isomer (AC 222293)

W. arylanilide herbicides such as 1-methylethyl-N-benzoyl-N-(3-chloro-4-fluorophenyl)-L-alanine (flamprop-isopropyl), ethyl N-benzoyl-N-(3,4-dichlorophenyl)-DL-alaninate (benzoylprop-ethyl), N-(2,4-difluorophenyl)-2-(3-(trifluoromethyl)phenoxy)-3-pyridinecarboxamide (diflufenican); and

X. amino acid herbicides such as N-(phosphonomethyl)glycine (glyphosate) and DL-homoalanin-4-yl-(methyl)phosphinic acid (phosphinothricin) and their salts and esters; and

Y. organoarsenical herbicides such as monosodium methanearsonate (MSMA); and

Z. miscellaneous herbicides including 4-(2,4-dichlorobenzoyl)-1,3-dimethyl-5-pyrazoly-p-toluene sulphonate (pyrazolate), (+)-2-(3,5-dichlorophenyl)-2-(2,2,2-trichloroethyl)oxirane (tridiphane), N,N-dimethyl-diphenylacetamide (diphenamid), N-(1-naphthyl)-phthalamic acid (naptalam) and 3-amino-1,2,4-triazole, 2-ethoxy-2,3-dihydro-3,3-dimethylbenzofuran methanesulfonate (ethofumesate), 1,4-epoxy-p-meth-2-yl 2-methylbenzyl ether (cinmethylin);

AA. Examples of useful contact herbicides include :

bipyridylium herbicides such as those in which the active entity is the 1,1′-dimethyl-4,4′-dipyridylium ion (paraquat) and those in which the active entity is the 1,1′-ethylene-2,2′-dipyridylium ion (diquat);

The following example illustrates the invention.

EXAMPLE 1

This Example illustrates the preparation of compound 1 in Table 1.

Step a

2-dichloromethyl-4-chloro phenyl isocyanate (111 g) obtained as described in Synthesis, 1978, 376-377, was dissolved in dry toluene (460 ml) and ammonia was bubbled through the solution with stirring for two hours, the temperature being maintained at from 15-20°C throughout. The reaction mixture was then filtered and the solid product washed with toluene. After further filtration, the solid 2-dichlorophenyl-4-chloro phenyl urea obtained was dried in a dessicator under reduced pressure.
Yield 117.5 g (99%).

Step b

The product from step a (78 g) was suspended in dioxan (390 ml) and cooled in an ice/water bath. 0.88 ammonia solution (177 ml) was added to the suspension with constant stirring. After the initial exothermic reaction had subsided, the ice/water bath was removed and the reaction mixture was stirred for a further 1¾ hours at a temperature of from 25-30°C. After heating to a temperature of 70-80°C for a further 1 hour, the reaction mixture was poured into water (1.5 litres) and filtered under reduced pressure. The solid residue was washed with a little water and then with methanol (2 x 100 ml). After drying in air, followed by drying under reduced pressure, 6-chloro-quinazolone was obtained (40 g, 72%).

Step c

The product from step b (39.63 g) was mixed with phosphoryl chloride (350 ml) and dimethylformamide (0.5 ml) and stirred under reflux for 5 hours (after 4 hours the solid material had dissolved). The reaction mixture was evaporated to dryness under reduced pressure and the solid residue triturated with water.

The product, 2,6-dichloroquinazoline was filtered under reduced pressure, air dried and dried further under reduced pressure (38 g).

Step d

The product from step c (1 g, 0.005 m), p-N-methylamino-phenol sulphate (0.87 g, 0.0025 m) and dry dimethylformamide (8.5 ml) were mixed together and stirred at 100°C for 1 hour. The reaction mixture was cooled, poured into water (50 ml) and extracted into ethyl acetate. The organic layer was washed with water, dried over magnesium sulphate, filtered and all traces of solvent removed under reduced pressure. 4-[N-(6-chloroquinazolin-2-yl)-N-methylamino]phenol was obtained as a bright yellow solid (1.02 g, 71%).

Step e

The product from step d (0.92 g) was stirred and refluxed in dry toluene (35 ml) for 15 minutes and then cooled until solid material began to precipitate out of the solution. 50% sodium hydride (0.15 g) was then added with stirring and the mixture stirred under reflux for a further 1 hour. After cooling, L-ethyl lactate mesylate (0.63 g) was added and the reaction mixture stirred under reflux for 7 hours. The solvent was removed under reduced pressure and the residue shaken with ethyl acetate/water. The organic extract was washed with water, dried over magnesium sulphate, filtered and the solvent removed from the filtrate under reduced pressure. After purification using column chromatography (Woelm silica/$CHCl_3$:$Et_2O$, 94:6) Compound 1 in Table 1 was obtained as a yellow oil (0.87 g, 70%).

EXAMPLE 2

This Example illustrates the preparation of Compound 2 in Table 1.

Step a

2-chloro-6-fluoro quinazoline was prepared using methods analogous to those described in Example 1 steps (a) to (c) except that (2-dichloromethyl-6-fluoro)phenyl isocyanate was employed in step a.

Step b

10 g (0.029 m) of p-N-methylaminophenol was stirred with water (15 ml) and anhydrous sodium acetate (4.76 g, 0.058 m) added followed by acetic anhydride (5.5 ml, 0.058 m). Stirring became difficult and so another portion (15 ml) of water was added. The reaction was stirred with gentle warming for 30 minutes,

cooled to room temperature and the solid product filtered off. The product, 4-hydroxy-N-methylacetanilide,was recrystallised from 95% ethanol, air dried and then dried under reduced pressure (4 g, 42%).

This step was repeated on a 10 fold scale to yield 77.74 g (81.1%) of product.

Step c

8.92 g (0.388 m) of sodium metal was dissolved in ethanol (1.3 l) and the solution cooled to room temperature. The product from step b (64.07 g) was added with stirring and with cooling in a water bath. After the addition, the reaction mixture was stirred at 50 °C for 15 minutes and the solvent removed under reduced pressure. The residue was azeotroped with toluene (2 x 325 ml) using a rotary evaporator under reduced pressure. A further portion of toluene (325 ml) was added to the reaction mixture together with L-ethyl lactate mesylate (76.10 g, 0.388 m). The reaction mixture was stirred under reflux for 6 hours, the solvent removed and the residue shaken with water/ethyl acetate. The aqueous phase was extracted with two further portions of ethyl acetate. The organic extracts were combined and washed three times with water. The organic phase was passed through a phase separating paper and the solvent removed from the filtrate under reduced pressure. The oil was dissolved in diethylether (300 ml) and stored at depressed temperatures for two days. The solid formed was filtered off under reduced pressure, and found to be the starting 4-hydroxy-N-methyl acetanilide.

The desired product

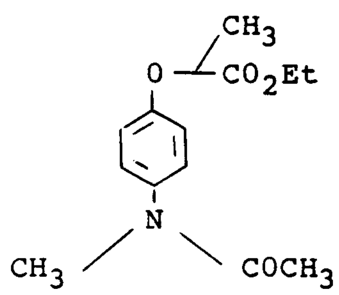

in the form of the D isomer, was obtained as a pale brown oil (94.62 g 92%), by evaporation of the filtrate under reduced pressure.

Step d

The product from step c (94.62 g, 0.356 m) was dissolved in ethanol (630 ml) and concentrated hydrochloric acid (250 ml) was added with constant stirring. The reaction was stirred under reflux for a total of 26 hours. The solvent was then removed from the reaction under reduced pressure. The residue was dissolved in water, neutralised with saturated sodium bicarbonate solution and extracted with three successive portions of ethyl acetate. The organic extract was washed three times with water, dried over $MgSO_4$, filtered and the solvent removed from the filtrate under reduced pressure. The product

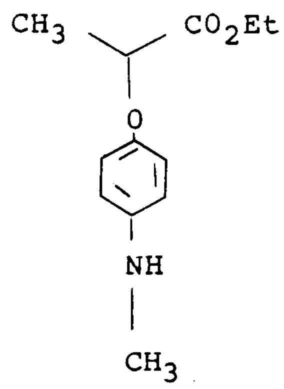

in the form of the D isomer, was obtained as a straw coloured oil (26.7 g, 33.5%)

Step e

The product from step d (24.5 g) and 2-chloro-6-fluoroquinazoline (20 g) were stirred under reflux in dry diglyme (120 $cm^3$) for 30 minutes. The solvent was removed from the reaction under reduced pressure. The residue was shaken with ethyl acetate/water. The aqueous phase was discarded and the organic extract washed with several successive portions of water/brine. The organic extract was dried over $MgSO_4$, filtered and the solvent removed from the filtrate under reduced pressure. The residue was a brown oil, which was triturated with cyclohexane and left to stand overnight. The following day most of the cyclohexane had evaporated leaving a moist solid. The solid was broken up and stirred with 40-60 petrol. The solid was filtered off, slurried with more 40-60 petrol in the sinter and the petrol pumped off. The product (Compound 2 in Table 1) was air dried. Yield = 34.56 g (85%).

EXAMPLE 3

This Example illustrates the preparation of Compound No 10 in Table 1.

Step a

A solution/suspension of N-chlorosuccinimide (9.05 g) in dichloromethane (350 ml) was added dropwise to a stirred solution of p-trifluoromethyl aniline (10.92 g) and 1,3-dithiane (8.15 g) in dichloromethane (150 ml) at a temperature of from -25°C to-20°C. The reaction was stirred at -20°C for 2 hours and left overnight at -20°C after which time a white solid had formed. A solution of NaOH (20 g) in water (400 ml) was added dropwise at a temperature of 0 to 5°C with vigorous stirring. The cooling bath was removed and stirring continued for 30 minutes. The layers were separated, the dichloromethane solution dried over magnesium sulphate for 15 minutes, filtered and the solvent removed using a rotary evaporator. The solid product was washed with 30-40° petrol (2 x 200 ml) and a minimum of chilled diethyl ether, and air-dried. The desired product,

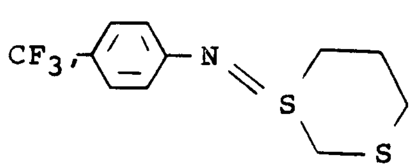

was obtained as a white solid (14.52 g, 77%).

Step b

The product from step a (9 g) was dissolved in n-propylamine (90 ml) and refluxed for 4 hours. The n-propylamine was removed using a rotary evaporator and analysis of the product by gas chromatography

showed it to be a mixture of anilines of formula

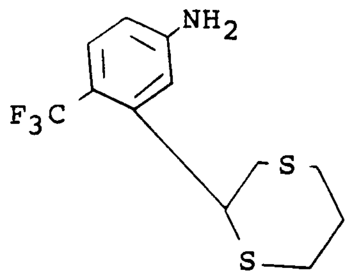

and

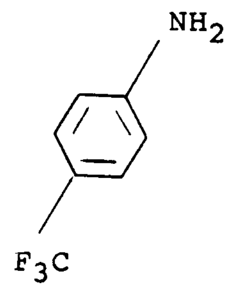

(i) (ii)

in the ratio 82:18

The mixture of anilines was separated from fast running bands and baseline material by column chromatography (HPLC, chloroform: Hexane 1:1, 30 ml min). The desired aniline (i) crystallised and the unwanted aniline (ii) was removed by washing with 30-40° petrol. Yield 5.5 g, 61%. Melting Point 96-7°C.

Step c

A solution of trichloroacetylchloride (3.21 g) in dichloromethane (20 ml) was added dropwise to a stirred solution of the product (i) of step b (4.70 g) in dichloromethane (50 ml) with ice/water bath cooling to a temperature of from 5° to 10°C. A solution of a triethylamine (1.79 g) in dichloromethane (20 ml) was added dropwise at a temperature of 5° to 10°C and the cooling bath removed. After 3½ hours, water (40 ml) was added, the mixture well stirred, the layers separated, the organic layer washed with water (40 ml), dried ($MgSO_4$), filtered and stripped. The remaining solid was washed with a minimum of cold petrol (30-40°C) and air-dried to yield the desired product

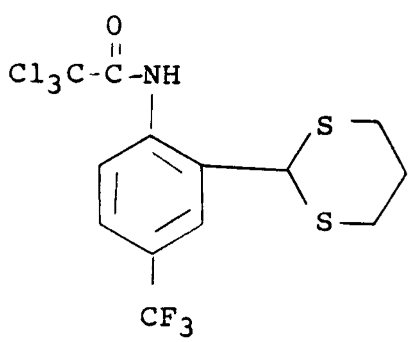

(6.7 g, 94%). Melting point 120-1°.

Step d

A solution of the product from step c (5.95 g) in tetrahydrofuran (30 ml) was added dropwise to a stirred mixture of red HgO (6.06 g, 2eq), $BF_3.OEt_2$ (4.17 g, 2.1 eq), tetrahydrofuran (50 ml) and water (10 ml), under argon. After 1 hour at ambient temperature and 1 hour at 50°C, some red colour remained. The $BF_3$ $OEt_2$ content was increased to 3 equivalents and after a further ½ hour at 50°C, the amount of water was gradually increased to 100 ml over ½ hour whereupon the red colour disappeared. The HgO content was increased to 3 equivalents and after ½ hour, thin layer chromatography showed that the reaction was complete. The reaction mixture was cooled to room temperature and stirred with 10% aq $Na_2CO_3$ (70 ml) for 10 minutes. The mixture was filtered through Hy-flow, washed through with ether (3 x 50 ml), the aqueous phase extracted with ether (2 x 50 ml), the combined organic layers washed with water (50 ml),

dried over $MgSO_4$, filtered and the solvents stripped using a rotary evaporator. The product

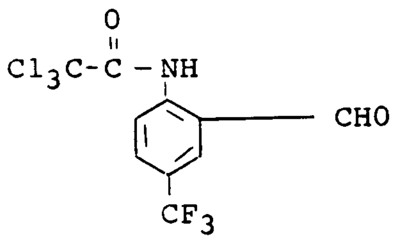

was obtained as a yellow solid. (4.28 g, 91%).

Step e

Following recrystallisation from hexane, the product from step d (3.5 g) was dissolved together with $NH_4OAc$ (5 g) in dimethylsulphoxide (DMSO) (40 ml) and the solution stirred over the weekend at room temperature and for 2 hours at 70 °C. The DMSO was distilled off under reduced pressure and the residue was triturated with water, filtered, washed with water (2 x 10 ml), acetone (2 x 10 ml), ethyl acetate (10 ml), diethyl ether (10 ml) and air-dried. The solid was left in an evacuated desiccator containing silica gel, overnight.

The product

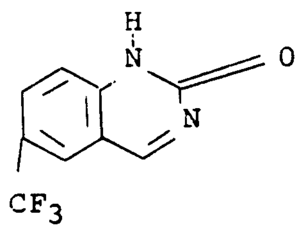

was obtained in 84% yield (1.88 g).

Step f

The product from step e (1.84 g) was dissolved in hot phosphoryl chloride (20 ml) and the solution heated under reflux for 3 hours. Dilution of the phosphoryl chloride solution with $CH_2Cl_2$ showed little sign of precipitation indicating that little starting material remained. The phosphoryl chloride was stripped using a rotary evaporator and water (20 ml) addede with ice-bath cooling. The mixture was neutralised by addition of solid $NaHCO_3$ with vigorous stirring. Dichloromethane (20 ml) was added, the mixture well shaken and the layers separated. The aqueous layer was filtered through a number 4 sinter and the organic layer through an extraction thimble. The aqueous layer was extracted with $CH_2Cl_2$ (2 x 20 ml), the combined organic solutions dried ($MgSO_4$), filtered and $CH_2Cl_2$ removed. Scratching gave the desired product.

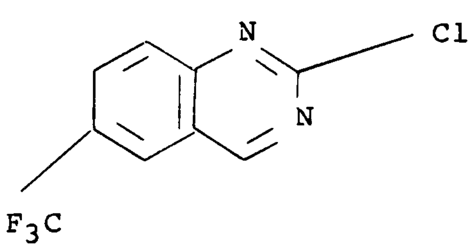

as a brown solid (1.60 g 80%).

Step g

A solution of the product from step (f) (197 mg) and ethyl-4-(N-methylamino)phenoxy propionate (189 mg) in diglyme (4 ml) was refluxed for 1 hour. The reaction was allowed to cool to room temperature, saturated aqueous $NaHCO_3$ (2 ml) and water (20 ml) added, and the mixture well stirred for 5 minutes. The mixture was filtered and the gum washed with water (3 x 5 ml). The gum was taken up in diethyl ether (20 ml), dried ($MgSO_4$), filtered and the diethyl ether removed using a rotary evaporator. The remaining gum was purified by column chromatography (13" x ½", 40-63 m silica, eluted with $CHCl_3$ 9: $Et_2O$ 1) to give Compound 10 of Table 1 (249 mg, 70%, 97% pure).

EXAMPLE 4

This Example illustrates the preparation of Compound No 13 in Table 1.

Step a

6-bromo-quinazolone (40 g), prepared as described in Rec Trav Chim 80 (1961) 149-57, and $POCl_3$ - (400 ml) were stirred together and heated to 110-120 °C in the presence of dimethylformamide (1 ml). After ½ hour, the solid dissolved to give a clear, dark solution which was heated for a further 2 hours and then excess $POCl_3$ was removed by distillation. The residue was added to ice water and stirred at room temperature to give a brown precipitate. This solid was filtered off and washed with water, air dried and then dried under reduced pressure to give ca 60 g of brown solid.

The procedure was repeated using 29 g of 6-bromoquinazolone and 300 ml of $POCl_3$ to yield a further 40 g of brown solid. The solids were combined and soxhlet extracted into 100-120 petrol. Evaporation of the solvent gave a yellow solid (13.6 g). The solid remaining in the soxhlet was washed several times with hot ethyl acetate. Evaporation of the combined washings gave 17.5 g of orange solid. The solids (13.6 g and 17.5 g) were combined and after recrystallisation from 100-120 petrol, 2-chloro-6-bromoquinazoline was obtained (28.54 g, 38%).

Step b

The product from step (a) (1.51 g), racemic ethyl 4-N-methylaminophenoxy propionate (1.4 g) and dry diglyme (10 ml) were refluxed together for about 70 minutes. The solvent was removed under reduced pressure and ethyl acetate added to the residue. The organic phase was then washed (4 x NaCl solution), and dried using $MgSO_4$, filtered and evaporated to a dark brown oil. This was purified using column chromatography (200 g silica; $CHCl_3$,95: $Et_2O$5) and the main bright yellow band collected. The eluent was filtered to remove any $SiO_2$ and the solvent removed under reduced pressure.

Drying under reduced pressure gave Compound 13 (2.22 g, 83%).

EXAMPLE 5

This Example illustrates the preparation of Compound No 12 of Table 1

Compound No 13 from Example 4 (0.6 g) was dissolved in a mixture of tetrahydrofuran (3 ml) and 2-propanol (15 ml) and sodium hydroxide (0.06 g) in water (3 ml) added. The mixture was stirred for 1½ hours at room temperature and the solvents removed under reduced pressure at 40 °C. Water was added to the residue which was then acidified with dilute hydrochloric acid. A yellow precipitate formed which was filtered off, air dried and dried further under reduced pressure. Compound No 12 was obtained as an orange glass (93% yield).

EXAMPLE 6

This Example illustrates the preparation of Compound No 6 in Table 1.

Step a

Sodium borohydride (5.95 g) was added to dry diglyme (100 ml) and stirred at room temperature for 10 minutes. 5-methyl-anthranilic acid (15.2 g) in dry diglyme (95 ml) was added dropwise with the reaction mixture being maintained at a temperature of from 15-25 °C, and stirred for 10 to 15 minutes. Prepowdered

aluminium chloride (7 g) was dissolved in dry diglyme (45 ml) at 0 to 5 °C and this solution was then added dropwise to the reaction mixture which was maintained at a temperature of from 20-35 °C. The resulting bright yellow suspension was stirred at room temperature for 1 hour and at 50 °C for a further 2 hours and then cooled to room temperature. The reaction mixture was then poured onto a mixture of ice (400 ml) and concentrated hydrochloric acid (12.5 ml) to give a final pH of approximately 6. After extraction with diethyl ether and ethyl acetate, the aqueous layer was basified with solid sodium bicarbonate and filtered. The filtrate was exhaustively extracted with diethyl ether and ethylacetate and the extracts were combined with those of the earlier extractions and washed with sodium chloride solution, sodium bicarbonate solution, again with sodium chloride solution and finally with water to remove diglyme and unreacted starting materials. The organic phase was then dried over magnesium sulphate and evaporated to give a brown oil which still contained some diglyme. As much of this as possible was removed under reduced pressure and the residue was then cooled until crystals formed. Hexane was added and the mixture filtered to give buff-coloured needle-like crystals (8 g). The filtrate was evaporated and further hexane added. Some additional crystals (2.5 g) were obtained in this way. Recrystallisation from toluene and hexane gave the desired product,

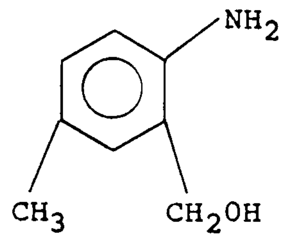

as fluffy beige needles (7.8 g; 54%) mp 122.5-122.8 °C.

Step b

Trichloroacetyl chloride (50 ml) was added to the product of step (a) (7.6 g) whereupon an exothermic reaction took place with HCl gas being evolved. The resulting dark liquid was heated under reflux for 1½ hours, cooled and the solvent removed under reduced pressure at 60 °C. The resulting brown solid was triturated with 30/40 petrol to give the desired product

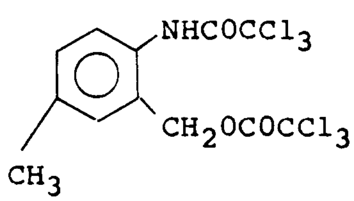

as a beige solid (16.5 g; 70%).

Step c

The product from step (b) (16.5 g) was dissolved in iso-propyl alcohol (200 ml) at 40 °C and sodium hydroxide (1.54 g) in water (15 ml) added dropwise. The solution became cloudy, then cleared and the clear solution was left for about 10 minutes before the solvent was removed under reduced pressure at a temperature of 40 °C. A brown oil was obtained which was purified by column chromatography (silica using ($CHCl_3$ 95: $Et_2O$ 5)). The main band was collected and evaporated to give the desired product

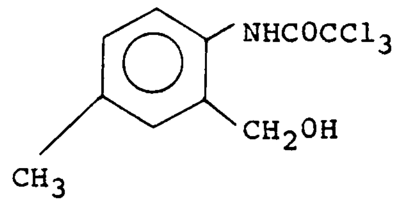

as a pale brown oil (8.5 g; 78%).

Step d

The product from step (c) (8.5 g) was dissolved in dry chloroform (300 ml) at room temperature and activated brown manganese dioxide (87 g) was added. After stirring for 1 hour, filtration through Hyflo, and washing through with dichloromethane, the filtrate was evaporated to give an orange oil which crystallised on standing. The desired product

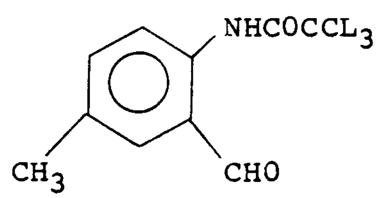

was obtained in 80% yield, (ca 80% pure)

Step e

The product from step (d) was cyclised using ammonium acetate in dimethylsulphoxide in a manner analogous to that described in Example 3 (e). The product

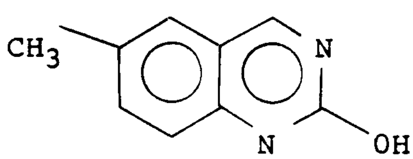

was chlorinated using phosphoryl chloride in a manner analogous to that described in Example 3 (f). The resulting compound

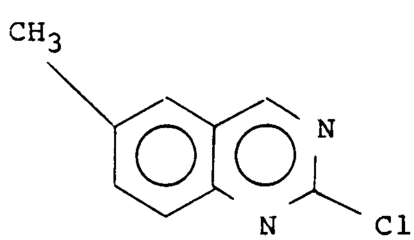

was coupled to the racemic ester

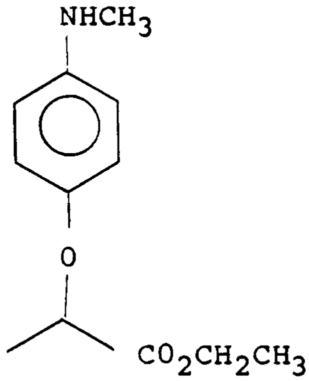

by a method analogous to that described in Example 3 (g) to give Compound 6.

EXAMPLE 7

Compounds 3-5, 7-9, 11 and 14 in Table 1 and Compounds 39 and 40 in Table 2 were obtained by methods analogous to those described in Examples 4 and 5.

EXAMPLE 8

This Example illustrates the preparation of Compound 15 in Table 1. Compound No 1 (1g) prepared as described in Example 1 was dissolved in butanol (50 $cm^3$) and concentrated sulphuric acid (0.22 $cm^3$) and stirred under reflux for 3 hours. Excess butanol was removed from the reaction vessel under reduced pressure . The residue was dissolved in ethyl acetate and washed several times with water. The organic extract was dried over $MgSO_4$, filtered and the solvent removed from the filtrate under reduced pressure. The residue was a yelow oil which was purified by chromatography using $CHCl_3$ : EtOAc, 97:3 to give the desired product.

EXAMPLE 9

Compounds 16-18 and 21 in Table 1 were prepared by methods analogous to these described in Example 8.

EXAMPLE 10

This Example illustrates the preparation of Compound 19 in Table 1.

Compound No 1 (4.93 g) prepared as described in Example 1 was dissolved in tetrahydrofuran (17 $cm^3$) and iso-propanol (48 $cm^3$). A solution of sodium hydroxide (0.52 g) in water (7.5 $cm^3$) was added slowly with constant stirring. The reaction mixture was stirred at room temperature for 1 hour and then the solvent was removed under reduced pressure at 40 ° C. The residue was dissolved in water (440 $cm^3$) and filtered. The filtrate was made slightly acid with 2M HCl and the solid formed filtered off, washed three times with water and air dried. Compound 19 was obtained as a yellow powder (3.58 g).

EXAMPLE 11

This Example illustrates the preparation of Compound 20 in Table 1.

Compound 19 (1 g) was dissolved in dry dichloromethane (10 $cm^3$) and propargyl alcohol (0.31 g) was added followed by 4-pyrolidinopyridine (0.04 g). The reaction mixture was cooled in a salt/ice bath and dicyclohexylcarbodiimide (0.63 g) added. After stirring with cooling for five minutes the reaction mixture was allowed to warm up to room temperature and stirred for a further 3 hours. The reaction mixture was diluted with dichloromethane then filtered to removed the urea formed in the reaction. The filtrate was washed once with diluted hydrochloric acid, then three times with water. The organic extract was dried over $MgSO_4$, filtered and the solvent removed from the filtrate under reduced pressure. The residue was purified by column chromatography ($CHCl_3$ : $Et_2O$, 95:5) to give the desired product.

EXAMPLE 12

Compounds 22-24 and 27-37 in Table 1 were prepared in a manner analogous to that described in Example 11.

EXAMPLE 13

This Example illustrates the preparation of Compound No 25 in Table 1. A solution of Compound 24 (3.2 g) in dry tetrahydrofuran (32 $cm^3$) was added slowly, with stirring and cooling to a suspension of methane sulphonamide (0.704 g) and sodium hydride (0.291 g) in dry tetrahydrofuran (16 $cm^3$). The reaction mixture was stirred at room temperature for $2\frac{1}{2}$ hours after which the solvent was removed under reduced pressure. The residue was then shaken with water and ethyl acetate and the aqueous layer separated, washed with ethyl acetate and acidified. The aqueous phase was then extracted with ethyl acetate and the organic extract washed three times with water, dried over magnesium sulphate, filtered and the solvent removed from the filtrate under reduced pressure. The solid residue was purified by flash chromatography using trichloromethane: ethyl acetate: acetic acid in the ratio 70:30:2 to give Compound 25 (1.7 g).

EXAMPLE 14

This Example illustrates the preparation of Compound 26 in Table 1.

Compound 19 (0.5 g) and triethanolamine (0.21 g) were dissolved in ethanol (10 $cm^3$) and stirred together for one hour. The solvent was then removed under reduced pressure and Compound 26 obtained as a yellow powder.

EXAMPLE 15

Compound 38 in Table I can be prepared from Compound 1 in Table I by methods analogous to those described in German Offenlegungsschrift No 3433390.

Biological Data

The herbicidal activity of the compounds was tested as follows:

Each compound in the appropriate concentration was incorporated into a 4% emulsion of methyl cyclohexanone and a 0.4% blend of 3.6 parts Tween 20 and 1 part Span 80. Tween 20 is a Trade Mark for a surface active agent comprising a condensate of 20 molar proportions of ethylene oxide with sorbitan laurate. Span 80 is a Trade Mark for a surface-active agent comprising sorbitan monolaurate. Formulation was effected by dissolving the compound in the requisite amount of solvent/surfactant blend. If necessary glass beads were added, the total liquid volume adjusted to 5 ml with water and the mixture shaken to effect complete dissolution of the compound. The formulation so prepared, after removal of beads where necessary, was then diluted to final spray volume (45 ml) with water.

The spray compositions so prepared were sprayed onto young pot plants (post-emergence test) at a rate equivalent to 1000 litres per hectare. Damage to plants was assessed 13 days after spraying by comparison with untreated plants, on a scale of 0 to 5 where 0 is 0-10% damage, 1 is 11 to 25% damage, 2 is 26-50% damage, 3 is 51-80% damage, 4 is 81-95% damage and 5 is 96-100% damage.

In a test carried out to detect pre-emergence herbicidal activity, seeds of the test species were placed on the surface of plastic trays of compost and sprayed with the compositions at the rate of 1000 litres per hectare. The seeds were then covered with further compost. 20 days after spraying, the seedlings in the sprayed plastic trays were compared with the seedlings in unsprayed control trays, the damage being assessed on the same scale of 0 to 5.

The results of the tests are given in Table 3 below.

TABLE 3

| COMPOUND NO | RATE OF APPLICATION kg/ha | PRE- OR POST-EMERGENCE APPLICATION | TEST PLANTS (see Table 4) | | | | | | | | | | | |
|---|---|---|---|---|---|---|---|---|---|---|---|---|---|---|
| | | | Mz | Ww | Rc | Br | Av | Dg | Al | St | Ec | Sh | Ag | Cn |
| 3 | 0.2 | Post | 5 | 4 | 3 | 5 | 4 | 5 | 4 | 5 | 5 | 5 | 5 | |
| 4 | 0.2 | Post | 5 | 4 | 3 | 5 | 4 | 5 | 3 | 5 | 5 | 5 | 3 | |
| 5 | 1.0 | Post | 2 | 3 | 3 | 4 | 4 | 5 | 4 | - | 5 | 4 | 3 | |
| 6 | 1.0 | Post | 4 | 2 | 5 | 4 | 3 | 4 | 4 | 5 | 5 | 5 | 4 | |
| 7 | 0.05 | Post | 3 | 1 | 1 | 3 | 0 | 2 | 4 | 3 | 5 | 4 | 2 | |
| 8 | 0.05 | Post | 3 | 3 | 2 | 4 | 2 | 4 | 4 | 4 | 5 | 4 | 4 | |
| 9 | 0.05 | Post | 3 | 2 | 0 | 4 | 0 | 3 | 3 | 4 | 5 | 4 | 3 | |

TABLE 3 CONTINUED

| COMPOUND NO | RATE OF APPLICATION kg/ha | PRE- OR POST-EMERGENCE APPLICATION | TEST PLANTS (see Table 4) | | | | | | | | | | | |
|---|---|---|---|---|---|---|---|---|---|---|---|---|---|---|
| | | | Mz | Ww | Rc | Br | Av | Dg | Al | St | Ec | Sh | Ag | Cn |
| 10 | 1 | Post | 5 | 2 | 0 | - | 0 | 4 | 3 | 4 | 5 | 5 | 2 | 1 |
| 11 | 2 | Pre | 4 | 5 | 4 | - | 4 | 5 | 4 | 5 | 5 | - | 5 | 2 |
| | | Post | 5 | 2 | 3 | - | 2 | 4 | 4 | 5 | 5 | 5 | 4 | 2 |
| 12 | 2 | Pre | 2 | 4 | 3 | - | 3 | 5 | 4 | 5 | 5 | 4 | - | 0 |
| | | Post | 5 | 4 | 2 | - | 1 | 4 | 3 | 4 | 5 | 4 | 4 | 0 |
| 13 | 2 | Pre | 3 | 5 | 4 | 4 | 4 | 4 | 4 | 5 | 4 | 4 | 5 | - |
| | | Post | 5 | 4 | 1 | 5 | 1 | 4 | 4 | 5 | 5 | 5 | 4 | - |
| 14 | 0.5 | Pre | 2 | 5 | 2 | - | 4 | 5 | 4 | 4 | 4 | 4 | 5 | 0 |
| | | Post | 4 | 4 | 3 | - | 2 | 4 | 4 | 4 | 5 | 5 | 4 | 0 |

TABLE 4

Abbreviations used for Test Plants

| | | |
|---|---|---|
| Mz | - | Maize |
| Ww | - | Winter Wheat |
| Rc | - | Rice |
| Br | - | Spring Barley |
| Av | - | *Avena fatua* |
| Dg | - | *Digitaria sanguinalis* |
| Al | - | *Alopercurus myosuroides* |
| St | - | *Setaria viridis* |
| Ec | - | *Echinochloa crus-galli* |
| Sh | - | *Sorghum halepense* |
| Ag | - | *Agropyron repens* |
| Cn | - | *Cyperus rotundus* |

The rice selectivity of certain compounds of the invention were demonstrated as follows:

Each compound in the appropriate concentration was incorporated into a 5% w/v emulsion of methyl cyclohexanone containing 10% w/v of a blend of Span 80 and Tween 20 in the ratio of 1:4. Complete dissolution was ensured by gentle warming at 20 ° C in a water bath.

The formulation was made up to final spray volume using 0.1% Agral 90 and sprayed post emergently onto various plants of a rate of 200/l/ha equivalent.

The plants sprayed and the growth stage at which they were sprayed are given in Table 4. The results of the spraying were assessed after 22 days on a 0-100 percentage scale where 0% was nil damage and 100% was complete kill. Table 5 shows the results of the tests.

TABLE 5

| TEST PLANTS | LEAF NUMBER |
|---|---|
| *Digitaria sanguinalis* (Dg) | 3 |
| *Echinochloa crus-galli* (Ec) | 4 |
| *Eleusine indica* (Ei) | 4 |
| *Sorghum halepense* (Sh) | 4 |
| *Setaria viridis* (St) | 4 |
| Rice cv Ishikari (Rc) | 3 |

TABLE 6

| COMPOUND NO | RATE OF APPLICATION g/ha | TEST PLANTS (see Table 5) | | | | | |
|---|---|---|---|---|---|---|---|
| | | Dg | Ec | Ei | Sh | St | Rc |
| 1 | 30 | 96 | 100 | 96 | 100 | 86 | 2 |
| | 60 | 100 | 100 | 97 | 100 | 99 | 3 |
| 17 | 60 | 99 | 100 | 92 | 100 | 75 | 1 |
| | 120 | 99 | 100 | 98 | 100 | 92 | 2 |

## Claims

**1.** A method for selectively controlling the growth of weeds in rice which process comprises applying to the rice or to the growth medium of the rice, a compound of formula (IIA):

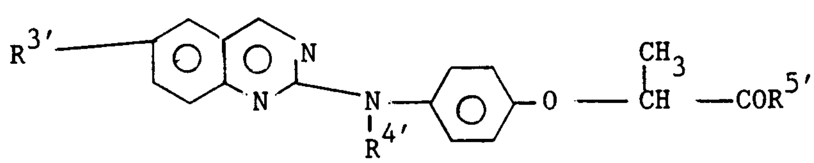

(IIA)

or an R-enantiomer thereof wherein $R^{3'}$ is halogen, $CF_3$ or methyl; $R^{4'}$ is methyl or ethyl; and $R^{5'}$ is $OR^6$ or $NHSO_2R^7$ wherein $R^6$ is hydrogen; $C_{1-10}$ alkyl, $C_{2-10}$ alkenyl or $C_{2-10}$ alkynyl, any of which may be optionally substituted by phenyl, $C_{1-10}$alkoxy, phenyl$C_{1-10}$alkoxy, phenoxy, cycloalkyl of up to 10 carbon atoms or tri$C_{1-10}$alkylsilyl; $C_{3-6}$ cycloalkyl; phenyl; p-nitrophenyl, p-chlorophenyl; 2,4-dichlorophenyl; or a cation and $R^7$ is $C_{1-10}$ alkyl or halo $C_{1-10}$ alkyl; in an amount sufficient to severely damage or kill the weeds but insufficient to damage the rice substantially.

**2.** A method according to claim 1 wherein $R^{4'}$ is methyl.

**3.** A method according to claim 1 or 2 wherein $R^{3'}$ is chloro.

**4.** A method according to any one of claims 1 to 3 wherein $R^{5'}$ is $OR^6$ and $R^6$ is $C_{1-6}$ alkyl.

**5.** A method according to any one of claims 1 to 3 wherein $R^{5'}$ is $-NHSO_2R^7$.

**6.** A method according to claim 5 wherein $R^6$ is ethyl or 2,2-(dimethyl) propyl.

**7.** A method according to claim 1 wherein the compound of formula (IIA) is a compound where $R^{3'}$ is chloro, $R^{4'}$ is methyl and $R^{5'}$ is ethoxy.

**8.** A method according to claim 7 wherein the compound of formula (IIA) is in the form of its R-enantiomer.

## Patentansprüche

**1.** Verfahren zur selektiven Bekämpfung des Wachstums von Unkräutern in Reisanpflanzungen, bei welchem auf die Reisanpflanzung oder auf das Wachstumsmedium der Reisanpflanzung eine Verbindung der Formel (II A)

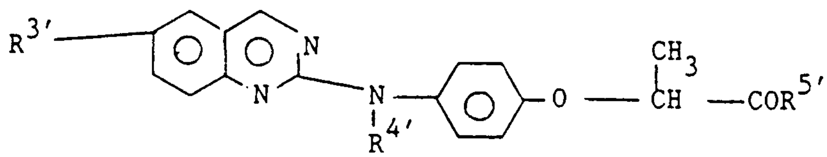

(IIA)

oder ein R-Enantionmer davon, worin $R^{3'}$ für Halogen, $CF_3$ oder Methyl steht, $R^{4'}$ für Methyl oder Ethyl steht und $R^{5'}$ für $OR^6$ oder $NHSO_2R^7$ steht, worin $R^6$ für Wasserstoff oder für $C_{1-10}$-Alkyl, $C_{2-10}$-Alkenyl oder $C_{2-10}$-Alkinyl, die jeweils durch Phenyl, $C_{1-10}$-Alkoxy, Phenyl-$C_{1-10}$-alkoxy, Phenoxy, Cycloalkyl mit bis zu 10 Kohlenstoffatomen oder Tri-$C_{1-10}$-alkylsilyl substituiert sein können, oder für $C_{3-6}$-Cycloalkyl, Phenyl, p-Nitrophenyl, p-Chlorophenyl, 2,4-Dichlorophenyl oder ein Kation steht und $R^7$ für $C_{1-10}$-Alkyl oder Halogeno-$C_{1-10}$-alkyl steht, in einer Menge aufgebracht wird, die ausreicht, die Unkräuter stark zu schädigen oder abzutöten, die aber nicht ausreicht, die Reispflanzen wesentlich zu schädigen.

**2.** Verfahren nach Anspruch 1, bei welchem $R^{4'}$ für Methyl steht.

**3.** Verfahren nach Anspruch 1 oder 2, bei welchem $R^{3'}$ für Chloro steht.

**4.** Verfahren nach einem der Ansprüche 1 bis 3, bei welchem $R^{5'}$ für $OR^6$ und $R^6$ für $C_{1-6}$-Alkyl steht.

**5.** Verfahren nach einem der Ansprüche 1 bis 3, bei welchem $R^{5'}$ für $-NHSO_2R^7$ steht.

**6.** Verfahren nach Anspruch 5, bei welchem $R^6$ für Ethyl oder 2,2-Dimethyl-propyl steht.

**7.** Verfahren nach Anspruch 1, bei welchem die Verbindung der Formel (II A) eine Verbindung ist, worin $R^{3'}$ für Chloro, $R^{4'}$ für Methyl und $R^{5'}$ für Ethoxy steht.

**8.** Verfahren nach Anspruch 7, bei welchem die Verbindung der Formel (II A) die Form des R-Enantiomers aufweist.

## Revendications

**1.** Procédé pour lutter sélectivement contre la croissance de mauvaises herbes dans les cultures de riz, procédé qui consiste à appliquer au riz ou au milieu de croissance du riz un composé de formule (IIA) :

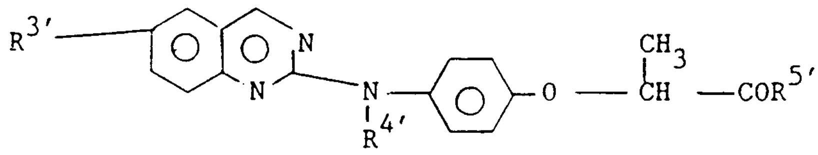

(IIA)

ou un énantiomère R d'un tel composé, formule dans laquelle $R^{3'}$ représente un halogène, un groupe $CF_3$ ou méthyle ; $R^{4'}$ représente un groupe méthyle ou éthyle ; et $R^{5'}$ représente un groupe $OR^6$ ou $NHSO_2R^7$ dans lequel $R^6$ représente l'hydrogène ; un groupe alkyle en $C_1$ à $C_{10}$, alcényle en $C_2$ à $C_{10}$ ou alcynyle en $C_2$ à $C_{10}$, dont l'un quelconque peut être facultativement substitué avec un groupe phényle, alkoxy en $C_1$ à $C_{10}$, phényl-(alkoxy en $C_1$ à $C_{10}$), phénoxy, cycloalkyle ayant jusqu'à 10 atomes de carbone ou tri(alkyle en $C_1$ à $C_{10}$)silyle ; cycloalkyle en $C_3$ à $C_6$ ; phényle ; p-nitrophényle, p-chlorophényle ; 2,4-dichlorophényle ; ou un cation et $R^7$ représente un groupe alkyle en $C_1$ à $C_{10}$ ou halogénalkyle en $C_1$ à $C_{10}$ ; en une quantité suffisante pour endommager gravement ou détruire les mauvaises herbes, mais insuffisante pour endommager le riz d'une manière notable.

**2.** Procédé suivant la revendication 1, dans lequel $R^{4'}$ représente un groupe méthyle.

**3.** Procédé suivant la revendication 1 ou 2, dans lequel $R^{3'}$ représente un groupe chloro.

**4.** Procédé suivant l'une quelconque des revendications 1 à 3, dans lequel $R^{5'}$ représente un groupe $OR^6$ et $R^6$ représente un groupe alkyle en $C_1$ à $C_6$.

**5.** Procédé suivant l'une quelconque des revendications 1 à 3, dans lequel $R^{5'}$ représente un groupe $NHSO_2R^7$.

**6.** Procédé suivant la revendication 5, dans lequel $R^6$ représente un groupe éthyle ou 2,2-(diméthyl)-propyle.

**7.** Procédé suivant la revendication 1, dans lequel le composé de formule (IIA) est un composé dans lequel $R^{3'}$ représente un groupe chloro, $R^{4'}$ représente un groupe méthyle et $R^{5'}$ représente un groupe éthoxy.

**8.** Procédé suivant la revendication 7, dans lequel le composé de formule (IIA) est sous forme de son énantiomère R.